# EUROPEAN PATENT APPLICATION

(11) **EP 4 424 808 A1**
(43) Date of publication of application: **04.09.2024**
(21) Application number: 22910055.7
(22) Date of filing: 21.12.2022
(51) Int. Cl.: C12M 1/00, C12M 1/42

(54) **TUBE ASSEMBLY**

(30) Priority: 21.12.2021 CN 202111572888
(71) Applicant: Nanjing GenScript Biotech Co., Ltd., Nanjing, Jiangsu 211100 (CN)
(72) Inventor: ZHANG, Hao, Nanjing, Jiangsu 211100 (CN); WANG, Lin, Nanjing, Jiangsu 211100 (CN); SHI, Gaofeng, Nanjing, Jiangsu 211100 (CN); QIAN, Hong, Nanjing, Jiangsu 211100 (CN)
(74) Representative: Wang, Bo
(86) International application number: PCT/CN2022/140570
(87) International publication number: WO 2023/116743

(57) **Abstract**

The embodiments of the present description provide a tube assembly. The tube assembly comprises fluid tubes and a mounting plate at least used for mounting the fluid tubes, the fluid tubes at least comprising a first tube, a second tube and a third tube. The first tube comprises a first tube input port and a first tube output port, wherein the first tube input port at least comprises a first tube first input sub-port and a first tube second input sub-port, and the first tube output port at least comprises a first tube first output sub-port and a first tube second output sub-port. The second tube comprises a second tube input port and a second tube output port. The third tube comprises a third tube input port and a third tube output port, wherein the third tube output port comprises a third tube first output sub-port, a third tube second output sub-port, a third tube third output sub-port and a third tube fourth output sub-port. The tube assembly is compact in structure, and thus is convenient and quick to install.

## Description

The present application claims priority to Chinese Patent Application No. 202111572888.2, filed on December 21, 2021, which is incorporated herein by reference in its entirety.

### Technical Field

This specification relates to the technical field of cell sorting, and particularly to a tube assembly.

### Background Art

In clinical medicine or life sciences, cell sorting, as key technology, can be applied to cell therapy, cell modification, cell culture, or the like. Specifically, cells with specific functions in a sample can be sorted by cell sorting technology to be used for cell therapy or cell modification, cell culture and the like.

A cell sorter is an essential device for cell sorting. The cell sorter is generally provided with a tube assembly for cell sorting. However, the tube assembly is complex. During mounting of the tube assembly, technicians often need to mount a plurality of tubes in sequence to the cell sorter and connect the tubes to one another to form the tube assembly. This process is time-consuming and labor-intensive, and involves the risks of human mistakes and mounting errors.

Therefore, how to quickly and accurately mount the tube assembly to the cell sorter has become an urgent problem to be solved at present.

### Summary

Embodiments of this specification provide a tube assembly, characterized in that the tube assembly comprises fluid tubes and a mounting plate for mounting at least the fluid tubes; wherein the fluid tubes comprise at least a first tube, a second tube, and a third tube, wherein the first tube comprises a first tube input port and a first tube output port, the first tube input port comprising at least a first tube first input sub-port and a first tube second input sub-port, and the first tube output port comprising at least a first tube first output sub-port and a first tube second output sub-port; the second tube comprises a second tube input port and a second tube output port; and the third tube comprises a third tube input port and a third tube output port, the third tube output port comprising a third tube first output sub-port, a third tube second output sub-port, a third tube third output sub-port, and a third tube fourth output sub-port.

In some embodiments, the fluid tubes further comprise a fourth tube, two ends of the fourth tube being connected to the first tube first output sub-port and the third tube second output sub-port, respectively.

In some embodiments, the tube assembly further comprises a sorting vessel, a first open end and a second open end of the sorting vessel being connected to the second tube output port and the third tube input port, respectively.

In some embodiments, the tube assembly further comprises a peristaltic hose, two ends of the peristaltic hose being connected to the first tube second output sub-port and the second tube input port, respectively.

In some embodiments, the tube assembly comprises at least one fluid supply component connected to the first tube input port of the first tube.

In some embodiments, the at least one fluid supply component comprises a first fluid supply component and a second fluid supply component; and the fluid tubes further comprise a fifth tube and a six tube; wherein two ends of the fifth tube are connected to the first fluid supply component and the first tube first input sub-port, respectively; and two ends of the sixth tube are connected to the second fluid supply component and the first tube second input sub-port, respectively.

In some embodiments, the tube assembly comprises at least one fluid receiving component connected to the third tube output port of the third tube.

In some embodiments, the at least one fluid receiving component comprises a first fluid receiving component, a second fluid receiving component, and a third fluid receiving component; and the fluid tubes further comprise a seventh tube, an eighth tube, and a ninth tube; wherein two ends of the seventh tube are connected to the first fluid receiving component and the third tube fourth output sub-port, respectively; two ends of the eighth tube are connected to the second fluid receiving component and the third tube third output sub-port, respectively; and two ends of the ninth tube are connected to the third fluid receiving component and the third tube first output sub-port, respectively.

In some embodiments, the tube assembly further comprises a filter vessel connected to the second tube output port.

In some embodiments, the second tube output port comprises a second tube first output sub-port, a second tube second output sub-port, and a second tube third output sub-port; and the fluid tubes further comprise a tenth tube and an eleventh tube; wherein two ends of the tenth tube are connected to the second tube first output sub-port and a first open end of the filter vessel, respectively; two ends of the eleventh tube are connected to a second open end of the filter vessel and the second tube second output sub-port, respectively; and the second tube third output sub-port is connected to the first open end of the sorting vessel.

In some embodiments, the second tube output port further comprises a second tube fourth output sub-port and a second tube fifth output sub-port; the first tube output port further comprises a first tube third output sub-port; the third tube output port further comprises a third tube fifth output sub-port; and the fluid tubes further comprise a twelfth tube and a thirteenth tube; wherein two ends of the twelfth tube are connected to the second tube fourth output sub-port and the first tube third output sub-port, respectively; and two ends of the thirteenth tube are connected to the second tube fifth output sub-port and the third tube fifth output sub-port, respectively.

In some embodiments, the tube assembly further comprises a fourth fluid receiving component; an eleventh tube output port is provided in the eleventh tube; and the fluid tubes further comprise a fourteenth tube, wherein two ends of the fourteenth tube are connected to the fourth fluid receiving component and the eleventh tube output port, respectively.

In some embodiments, a plurality of mounting grooves are provided on one side of the mounting plate, and the plurality of mounting grooves are configured for fixedly mounting and/or guiding the fluid tubes.

In some embodiments, at least one of the plurality of mounting grooves is configured for fixedly mounting the filter vessel and/or the sorting vessel.

In some embodiments, the mounting plate is provided with a plurality of reinforcing ribs on a side opposite the mounting grooves.

In some embodiments, a plurality of valve holes are provided in the mounting plate, and are arranged in the plurality of mounting grooves.

In some embodiments, the tube assembly further comprises on-off valves disposed in the valve holes, a notch being provided at an end of each of the on-off valves facing an opening of the mounting groove and being configured for mounting a tube of the fluid tubes.

In some embodiments, the fluid tubes form a plurality of fluid paths between the sorting vessel and/or the filter vessel and the peristaltic hose, and each of the plurality of fluid paths passes through the sorting column and the peristaltic hose.

### Brief Description of the Drawings

The present application will be further illustrated by way of exemplary embodiments, and these exemplary embodiments will be described in detail with reference to the accompanying drawings. These embodiments are not limiting, and in these embodiments, the same numerals represent the same structures, in which:
FIG. 1 is a schematic structural diagram of a tube assembly according to some embodiments of this specification;
FIG. 2 is a schematic structural diagram of a first tube shown in FIG. 1;
FIG. 3 is a schematic structural diagram of a second tube shown in FIG. 1;
FIG. 4 is a schematic structural diagram of a third tube shown in FIG. 1;
FIG. 5 is a schematic structural diagram of a sorting vessel according to some embodiments of this specification;
FIG. 6 is a schematic structural diagram of a tube assembly comprising a filter vessel according to some embodiments of this specification;
FIG. 7 is a schematic structural diagram of a second tube of the tube assembly shown in FIG. 6;
FIG. 8 is a schematic structural diagram of a first tube of the tube assembly shown in FIG. 6;
FIG. 9 is a schematic structural diagram of a third tube of the tube assembly shown in FIG. 6;
FIG. 10 is a schematic structural diagram of a tube assembly according to some embodiments of this specification;
FIG. 11 is a front schematic structural diagram of a mounting plate of the tube assembly shown in FIG. 10; and
FIG. 12 is a back schematic structural diagram of the mounting plate of the tube assembly shown in FIG. 10.

### Detailed Description of Embodiments

For a clearer description of the technical solutions in the embodiments of the present application, a brief introduction will be given below for the accompanying drawings required for describing the embodiments. Apparently, the accompanying drawings in the following description show merely some embodiments of the present application, and those of ordinary skill in the art may still apply the present application to other similar scenarios according to these accompanying drawings without any creative effort. Unless obvious from the linguistic context or otherwise stated, the same reference signs in the drawings represent the same structure or operation.

It should be understood that "system", "device", "unit" and/or "module" as used herein is a method for distinguishing different components, elements, parts, portions or assemblies of different levels. However, these words may be substituted by other expressions if these expressions accomplish the same purpose.

As shown in the present application and the claims, the words "one", "a", "an" and/or "the" do not specifically refer to the singular, but may also include the plural, unless the context clearly indicates otherwise. Generally, the terms "including" and "comprising" only imply the inclusion of explicitly identified steps and elements, and these steps or elements do not constitute an exclusive list. A method or a device may further comprise other steps or elements.

With the rapid development of life sciences and medicine in recent years, the cell sorting technology has been widely used in medical or experimental scenarios such as cell therapy, cell modification or cell culture. Generally, cell sorting needs to be implemented by means of a cell sorter.

In cell sorting using the cell sorter, a cell sorting process may include multiple steps, such as sorting target cells by means of a sorting vessel, releasing the target cells in a sorting instrument, collecting the target cells, and rinsing tubes prior to cell sorting and/or posterior to a corresponding step (e.g., sorting target cells by means of a sorting vessel). Here, the target cells are cells with some specific functions in a sample that need to be sorted out. For example, the target cells may include, but are not limited to, tumor stem cells, peripheral blood cells, hematopoietic stem cells, neural cells, etc.

A tube assembly of the cell sorter is one of essential parts for cell sorting. The tube assembly of the cell sorter can create a plurality of fluid paths for implementing various steps of the cell sorting process, respectively.

In some embodiments, the cell sorter is designed separately from the tube assembly thereof, that is, the tube assembly needs to be mounted to the cell sorter before cell sorting is performed. The tube assembly comprises at least a plurality of tubes. During mounting, the plurality of tubes need to be manually mounted to the cell sorter in sequence, and the plurality of tubes need to be connected to one another and to a sorting vessel and/or components for storing and/or buffering a fluid (e.g., a sample fluid for cell sorting, a buffer solution for tube rinsing, a waste fluid after tube rinsing, a solution that contains only target cells, and a solution that contains no target cell). The entire mounting is tedious, time-consuming and labor-intensive, and is prone to the risks of human mistakes and mounting errors.

Embodiments of this specification provide a tube assembly. The tube assembly comprises fluid tubes and a mounting plate for mounting at least the fluid tubes. The fluid tubes may comprise a plurality of tubes. The plurality of tubes may be mounted to the mounting plate, are connected to one another on the mounting plate, and are connected to other components (e.g., a sorting vessel, and a component for storing and/or buffering a fluid) for cell sorting, so as to provide conditions for forming fluid paths corresponding to the various steps of the cell sorting process. In some embodiments, the sorting vessel, the component for storing and/or buffering a fluid, etc. can be mounted to the mounting plate to be connected to the fluid tubes or can be connected to the fluid tubes independently of the mounting plate. By means of mounting the fluid tubes to the mounting plate to form an integrated assembly, and then connecting other components for cell sorting (e.g., the sorting vessel, and the component for storing and/or buffering a fluid) to the fluid tubes, or by means of mounting the fluid tubes, the sorting vessel, and the component for storing and/or buffering a fluid, etc. to the mounting plate and connecting them to each other to form an integrated assembly, only positioning and mounting of the cell sorter by means of the mounting plate are needed during cell sorting. For example, the tube assembly can be mounted to the cell sorter by means of fitting between positioning holes on the mounting plate and a positioning mechanism of the cell sorter. The tube assembly is compact in structure and is easy and quick to mount, and accordingly the time for manual mounting can be reduced and the risks caused during manual mounting, for example, improper mounting of the tube assembly, or failure to join the tubes of the tube assembly with a corresponding pump and valves due to incorrect mounting positions of the tubes on the cell sorter, can be eliminated.

The tube assembly provided in the embodiments of this specification will be described in detail below with reference to the accompanying drawings.

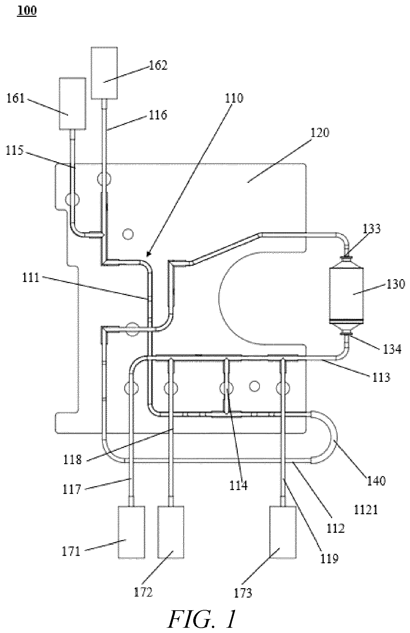

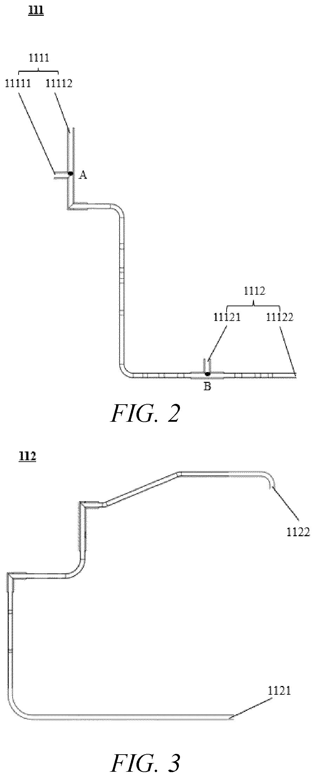

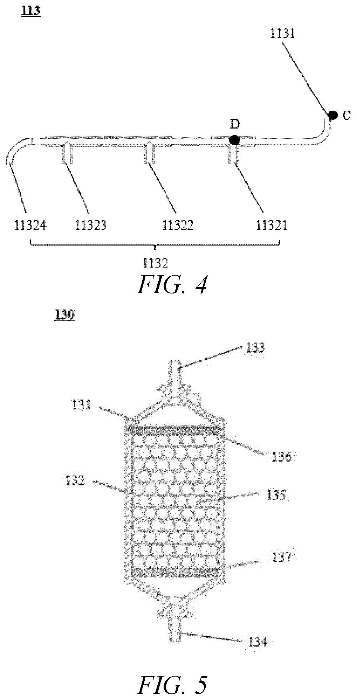

FIG. 1 is a schematic structural diagram of a tube assembly according to some embodiments of this specification. FIG. 2 is a schematic structural diagram of a first tube shown in FIG. 1. FIG. 3 is a schematic structural diagram of a second tube shown in FIG. 1. FIG. 4 is a schematic structural diagram of a third tube shown in FIG. 1.

As shown in FIG. 1, the tube assembly 100 may comprise fluid tubes 110 and a mounting plate 120 for mounting at least the fluid tubes 110. The fluid tubes 110 comprise at least a first tube 111, a second tube 112, and a third tube 113. In some embodiments, the first tube 111 may serve as a primary tube for fluid inflow during a cell sorting process. That is, during the cell sorting process, a fluid (e.g., a sample fluid and a buffer fluid) from fluid supply components (e.g., a first fluid supply component 161 and a second fluid supply component 162 in FIG. 1) flows to tubes of the tube assembly 100 mainly through the first tube 111. In some embodiments, the third tube 113 may serve as a primary tube for fluid outflow during the cell sorting process. That is, a fluid (e.g., a fluid that contains only target cells, a fluid that contains no target cell, and a buffer fluid after tube rinsing) in the cell sorting process flows mainly from the third tube and flows to corresponding fluid receiving components (e.g., a first fluid receiving component 171, a second fluid receiving component 172, and a third fluid receiving component 173 of FIG. 1). In some embodiments, the second tube 112 may be configured to connect the first tube 111 and the third tube 113 and form a plurality of fluid paths with the first tube 111 and the third tube 113 for cell sorting. In addition, the second tube 112 and the first tube 111 may be connected to each other via a peristaltic hose (e.g., a peristaltic hose 140). The peristaltic hose may be jointed to a peristaltic pump to supply power for the flow of the fluid in the fluid paths during the cell sorting process.

With reference to FIG. 1 and FIG. 2, the first tube 111 may comprise a first tube input port 1111 and a first tube output port 1112. The first tube input port 1111 may comprise at least a first tube first input sub-port 11111 and a first tube second input sub-port 11112; and the first tube output port 1112 may comprise at least a first tube first output sub-port 11121 and a first tube second output sub-port 11122.

With reference to FIG. 1 and FIG. 3, the second tube 112 may comprise a second tube input port 1121 and a second tube output port 1122.

With reference to FIG. 1 and FIG. 4, the third tube 113 may comprise a third tube input port 1131 and a third tube output port 1132. The third tube output port 1132 may comprise a third tube first output sub-port 11321, a third tube second output sub-port 11322, a third tube third output sub-port 11323, and a third tube fourth output sub-port 11324.

In some embodiments, when there are a plurality of input sub-ports and/or a plurality of output sub-ports in a tube of the fluid tubes, the plurality of input sub-ports and/or the plurality of output sub-ports may be formed by tube connectors mounted to an input end and/or an output end of a tube body.

Taking the first tube 111 as an example, a tee connector may be mounted at one end (e.g., the end close to the first tube input port 1111) of the tube body of the first tube 111. The tee connector has three tube joints. One of the three tube joints is connected to one end of the tube body of the first tube 111, and the other two tube joints may serve as the first tube first input sub-port 11111 and the first tube second input sub-port 11112. Similarly, a tee connector may be mounted at the other end (e.g., the end close to the first tube output port 1112) of the tube body of the first tube 111. One of the tube joints of the tee connector is connected to the other end of the tube body of the first tube 111, and the other two tube joints may serve as the first tube first output sub-port 11121 and the first tube second output sub-port 11122.

Taking the third tube 113 as another example, three tee connectors may be sequentially mounted at the output end of the tube body of the third tube 113, i.e., a first tee connector, a second tee connector, and a third tee connector is provided, from near to far, from one end (e.g., the end close to the third tube output port 1132) of the tube body of the third tube 113. The two tube joints of the first tee connector that form a straight channel are connected to the output end of the tube body of the third tube 113 and one of the two tube joints of the second tee connector that form a straight channel, respectively. The other of the two joints of the second tee connector that form the straight channel is connected to one of the two tube joints of the third tee connector that form a straight channel. In this way, the tube joint of the first tee connector that is not involved in the formation of the straight channel can act as the third tube first output sub-port 11321, the tube joint of the second tee connector that is not involved in the formation of the straight channel can serve as the third tube second output sub-port 11322, the tube joint of the third tee connector that is not involved in the formation of the straight channel can act as the third tube third output sub-port 11323, and the other of the two tube joints of the third tee connector that form the straight channel serves as the third tube fourth output sub-port 11324. In some embodiments, an additional tube may be provided between the output end of the tube body of the third tube 113, the first tee connector, the second tee connector and the third tee connector, such that the two tube joints that are originally directly connected to each other are indirectly connected to each other via the tube, so as to adjust a distance between the third tube first output sub-port 11321, the third tube second output sub-port 11322 and the third tube third output sub-port 11323 to satisfy actual mounting requirements. In some embodiments, instead of the three sequentially connected tee connectors, a five-way tube connector may be used to be mounted at the output end of the tube body of the third tube 113. The five-way tube connector comprises five tube joints, with two of the five tube joints forming a straight channel. One of the two tube joints that form the straight channel is connected to the output end of the tube body of the third tube 113, and the other of the two tube joints that form the straight channel acts as the third tube fourth output sub-port 11324. The other three tube joints of the third tube first output sub-port 11321, the third tube second output sub-port 11322 and the third tube third output sub-port 11323 that are not involved in the formation of the straight channel serve as the third tube first output sub-port 11321, the third tube second output sub-port 11322, and the third tube third output sub-port 11323, respectively.

In some embodiments, the tube body of the first tube 111 or the third tube 113 may be understood to be a portion of the first tube 111 or the third tube 113 that excludes the input port and the output port, or a portion between the input port (or one of the output sub-ports) and the output port (or one of the output sub-ports). For example, the tube body of the first tube 111 may be a portion of the first tube 111 from a junction A between the first tube first input sub-port 11111 and the first tube second input sub-port 11112 to a junction B between the first tube first output sub-port 11121 and the first tube second output sub-port 11122. As another example, the tube body of the third tube 113 may be a portion of the third tube 113 from a position C of the third tube input port 1131 to a position D corresponding to the third tube first output sub-port 11121.

In some embodiments, the first tube 111, the second tube 112, and the third tube 113 may be a single tube (e.g., an integrated multi-way tube). The tube itself has a plurality of tube joints, and the plurality of tube joints may act as the input sub-port and/or output sub-port of the first tube 111 and/or the third tube. In some embodiments, the tube has the form of the first tube 111, the second tube 112, and the third tube 113 that are connected. The tube comprises an input port and an output port. A tube joint is provided on the body of the tube close to the input port, and the tube joint and the input port may act as the first tube first input sub-port 11111 and the first tube second input sub-port 11112, respectively. In some embodiments, a tube joint is provided on a middle portion of the tube (i.e., the position corresponding to the first tube first output sub-port 11121), and the tube joint may act as the first tube first output sub-port 11121. In some embodiments, three tube joints are provided in sequence on the body of the tube close to the output port, and the three tube joints and the output port may act as the third tube first output sub-port 11321, the third tube second output sub-port 11322, the third tube third output sub-port 11323, and the third tube fourth output sub-port 11324, respectively.

With continued reference to FIG. 1, the fluid tubes 110 may further comprise a fourth tube 114, and two ends of the fourth tube 114 are connected to the first tube first output sub-port 11121 and the third tube second output sub-port 11322, respectively, thereby realizing a connection between the first tube 111 and the third tube 113.

In some embodiments, as shown in FIG. 1, the tube assembly 100 further comprises a sorting vessel 130, and a first open end and a second open end of the sorting vessel 130 can be connected to the second tube output port 1122 and the third tube input port 1131, respectively, thereby realizing a connection between the second tube 112 and the third tube 113.

In some embodiments, the sorting vessel 130 may not be included in the tube assembly 100, that is, the sorting vessel 130 may be connected to the tube assembly 100 and mounted to the cell sorter independently of the tube assembly 100. In some embodiments, the sorting vessel 130 may be connected to the tube assembly 100 and/or mounted to the cell sorter before or after the tube assembly 100 is mounted to the cell sorter. In some embodiments, the tube assembly 100 may additionally reserve two ports (e.g., the second tube output port 1122 and the third tube input port 1131) for connection with a first open end 133 and a second open end 134 of the sorting vessel 130 respectively.

In some embodiments, the sorting vessel 130 may be configured for adsorbing target cells to achieve the purpose of cell sorting. FIG. 5 is a schematic structural diagram of a sorting vessel according to some embodiments of this specification. As shown in FIG. 5, a housing of the sorting vessel 130 comprises an upper housing 131 and a lower housing 132. The upper housing 131 and the lower housing 132 are provided with the first open end 133 and the second open end 134, respectively. The upper housing 131 and the lower housing 132 may form a cavity which is filled with a plurality of ferromagnetic spheres 135. Before a sample enters the sorting vessel 130, a magnetic field needs to be applied to the sorting vessel 130 to create a high magnetic gradient within the sorting vessel 130, contributing to adsorbing the target cells in the sample within the sorting vessel 130. The target cells may be cells labeled with magnetic particles. After the magnetic field is removed, the high magnetic gradient within the sorting vessel 130 disappears, contributing to releasing the target cells adsorbed within the sorting vessel 130. In some embodiments, a first sieve plate 136 and a second sieve plate 137 are disposed at each of upper and lower ends of the cavity of the sorting vessel 130, and the plurality of ferromagnetic spheres 135 are located within the cavity space defined by the first sieve plate 136 and the second sieve plate 137 to prevent the ferromagnetic spheres 135 from falling out of the sorting vessel 130. In some embodiments, the first sieve plate 136 and the second sieve plate 137 are provided with micro pores to facilitate the flow of a fluid in the sorting vessel 130.

With continued reference to FIG. 1, the tube assembly 100 further comprises a peristaltic hose 140, and two ends of the peristaltic hose 140 are connected to the first tube second output sub-port 11122 and the second tube input port 1121, respectively, thereby realizing a connection between the first tube 111 and the second tube 112.

In some embodiments, the peristaltic hose 140 may not be included in the tube assembly 100, that is, the peristaltic hose 140 may be connected to the tube assembly 100 and mounted to the cell sorter independently of the tube assembly 100. In some embodiments, the peristaltic hose may be connected to the tube assembly 100 and/or mounted to the cell sorter before or after the tube assembly 100 is mounted to the cell sorter. In some embodiments, the tube assembly 100 may additionally reserve two ports (e.g., the first tube second output sub-port 11122 and the second tube input port 1121) for connection with two ends of the peristaltic hose 140, respectively.

In some embodiments, the peristaltic hose 140 may be jointed to the peristaltic pump to supply power for the flow of the fluid in cell sorting fluid paths formed by the tube assembly 100. In some embodiments, the peristaltic pump may be disposed on the cell sorter. When the tube assembly 100 is mounted to the cell sorter by means of the mounting plate 120, the peristaltic hose 140 can be operably jointed to the peristaltic pump, and power generated by the peristaltic pump can drive the fluid to flow along corresponding fluid paths through the peristaltic hose 140.

In some embodiments, the tube assembly 100 may further comprise at least one fluid supply component. The at least one fluid supply component may be connected to the first tube input port 1111 of the first tube to provide a sample fluid (e.g., a blood sample solution or culture fluid that contains target cells) that requires cell sorting and/or a buffer fluid (e.g., normal saline) for tube rinsing during the cell sorting process.

In some embodiments, the at least one fluid supply component may not be included in the tube assembly 100, that is, the at least one fluid supply component may be connected to the tube assembly 100 and mounted to the cell sorter independently of the tube assembly 100. In some embodiments, the at least one fluid supply component may be connected to the tube assembly 100 and/or mounted to the cell sorter before or after the tube assembly 100 is mounted to the cell sorter. In some embodiments, the tube assembly 100 may additionally reserve at least one port (e.g., the first tube first input sub-port 11111 and the first tube second input sub-port 11112) for connection with the at least one fluid supply component.

In some embodiments, with continued reference to FIG. 1, the at least one fluid supply component may comprise a first fluid supply component 161 and a second fluid supply component 162. The first fluid supply component 161 can be configured for storing and/or providing the sample fluid (e.g., the blood solution or culture fluid that contains target cells) that requires cell sorting. The second fluid supply component 162 can be configured for storing and/or providing the buffer fluid (e.g., normal saline) for tube raising.

In some embodiments, the fluid tubes 110 may further comprise a fifth tube 115 and a sixth tube 116. Two ends of the fifth tube 115 are connected to the first fluid supply component 161 and the first tube first input sub-port 11111 respectively such that the sample fluid in the first fluid supply component 161 may flow into the first tube 111 through the fifth tube 115 and the first tube first input sub-port 11111. Two ends of the sixth tube 116 are connected to the second fluid supply component 162 and the first tube second input sub-port 11112 respectively such that the buffer fluid in the second fluid supply component 162 may flow into the first tube 111 through the sixth tube 116 and the first tube second input sub-port 11112.

In some embodiments, the tube assembly 100 further comprise at least one fluid receiving component. The at least one fluid receiving component may be connected to the third tube output port 1132 of the third tube 113 (e.g., the third tube first output sub-port 11321, the third tube third output sub-port 11323, and the third tube fourth output sub-port 11324) to receive the sample fluid with the target cells sorted out and that flows from the sorting vessel 130 (e.g., the sample fluid that contains no target cell), the buffer fluid after tube rinsing, and the target cells or a fluid that contain only target cells after cell sorting.

In some embodiments, the at least one fluid receiving component may not be included in the tube assembly 100, that is, the at least one fluid receiving component may be connected to the tube assembly 100 and mounted to the cell sorter independently of the tube assembly 100. In some embodiments, the at least one fluid receiving component may be connected to the tube assembly 100 and/or mounted to the cell sorter before or after the tube assembly 100 is mounted to the cell sorter. In some embodiments, the tube assembly 100 may additionally reserve at least one port (e.g., the third tube first output sub-port 11321, the third tube third output sub-port 11323, or the third tube fourth output sub-port 11324) for connection with the at least one fluid receiving component.

In some embodiments, with continued reference to FIG. 1, the at least one fluid receiving component may comprise a first fluid receiving component 171, a second fluid receiving component 172, and a third fluid receiving component 173. The first fluid receiving component 171 may be configured for receiving the sample fluid (e.g., the sample fluid that contains no target cell) with the target cells sorted out and that flows from the sorting vessel 130. The second fluid receiving component 172 can be configured for receiving the buffer fluid (or waste fluid) after tube rinsing. The third fluid receiving component 173 can be configured for receiving (or collecting) the target cells or the fluid that contains only target cells after cell sorting.

In some embodiments, the fluid tubes 110 further comprise a seventh tube 117, an eighth tube 118, and a ninth tube 119. Two ends of the seventh tube 117 can be connected to the first fluid receiving component 171 and the third tube fourth output sub-port 11324 respectively such that the sample fluid that contains no target cell and flows from the sorting vessel 130 can flow into the first fluid receiving component 171 through the third tube fourth output sub-port 11324 and the seventh tube 117. Two ends of the eighth tube 118 can be connected to the second fluid receiving component 172 and the third tube third output sub-port 11323 respectively such that the buffer fluid after tube rinsing can flow into the second fluid receiving component 172 through the third tube third output sub-port 11323 and the eighth tube 118. Two ends of the ninth tube 119 can be connected to the third fluid receiving component 173 and the third tube first output sub-port 11321 respectively such that the target cells or the fluid that contains only target cells after cell sorting can flow into the third fluid receiving component 173 through the third tube first output sub-port 11321 and the ninth tube 119 for collection.

In some embodiments, the tube assembly 100 may comprise only two fluid receiving components. One of the fluid receiving components (e.g., the third fluid receiving component 173) is configured for receiving the third tube first output sub-port 11321, and the other fluid receiving component (e.g., the first fluid receiving component 171 or the second fluid receiving component 172) is configured for receiving the sample fluid that contains no target cell and flows from the sorting vessel 130 as well as the buffer fluid after tube rinsing. When the tube assembly 100 comprises only two fluid receiving components, one output sub-port (e.g., the third tube third output sub-port 11323 or the third tube fourth output sub-port 11324) can be omitted for the third tube output port 1132, and correspondingly, the seventh tube 117 or the eighth tube 118 can be dispensed with.

It should be noted that the terms "input port," "output port," "input sub-port," and "output sub-port" referred to in this specification are described only as names of ports and are not intended to limit the inflow or outflow direction of a fluid. In some embodiments, the "input port" or "input sub-port" may also be used as a port for fluid outflow, and the "output port" or "output sub-port" may also be used as a port for fluid inflow.

In some embodiments, in the tube assembly 100, the fluid tubes 110 form a plurality of fluid paths for cell sorting between the sorting vessel 130 and the peristaltic hose 140, and each of the plurality of fluid paths passes through a sorting column and the peristaltic hose for implementing the various steps of the cell sorting process. In some embodiments, the various steps of the cell sorting process are implemented with the fluid (e.g., the sample fluid and/or buffer fluid) flowing along corresponding fluid paths. The cell sorting process will be described in detail below with respect to the tube assembly 100 shown in FIG. 1.

In some embodiments, the cell sorting process based on the tube assembly 100 may include the following steps.

At step 1, the tubes are pre-rinsed. The pre-rinsing of the tubes mainly involves discharging air from the tubes (e.g., the first tube 111, the second tube 112, and the third tube 113) and/or the sorting vessel 130 by means of the buffer fluid in the second fluid supply component 162, and the buffer fluid after the rinsing flowing into the second fluid receiving component 172. In some embodiments, a fluid path corresponding to the tube pre-rinsing sequentially includes: the second fluid supply component 162, the sixth tube 116, the first tube second input sub-port 11112, the first tube 111, the first tube second output sub-port 11122, the peristaltic hose 140, the second tube input port 1121, the second tube 112, the second tube output port 1122, the first open end 133 of the sorting vessel 130, the sorting vessel 130, the second open end 134 of the sorting vessel 130, the third tube input port 1131, the third tube 113, the third tube third output sub-port 11323, the eighth tube 118, and the second fluid receiving component 172. That is, the pre-rinsing of the tubes is implemented by the buffer fluid filling the tubes and flowing through the tubes, such that the air in the tubes (e.g., the first tube 111, the second tube 112, and the third tube 113) and/or the sorting vessel 130 can be discharged.

At step 2, primary cell sorting is carried out. The primary cell sorting mainly involves allowing the sample fluid in the first fluid supply component 161 to flow into the sorting vessel 130, and applying a magnetic field to the sorting vessel 130, such that the target cells (e.g., the cells labeled with the magnetic particles) in the sample fluid can be adsorbed in the sorting vessel 130 under the action of the magnetic field, and the sample fluid that flows from the sorting vessel 130 is a sample fluid that contains no target cell, and then the sample fluid that contains no target cell flows into the first fluid receiving component 171. A fluid path corresponding to the primary cell sorting sequentially includes: the first fluid supply component 161, the first tube first input sub-port 11111, the first tube 111, the first tube second output sub-port 11122, the peristaltic hose 140, the second tube input port 1121, the second tube 112, the second tube output port 1122, the first open end 133 of the sorting vessel 130, the sorting vessel 130, the second open end 134 of the sorting vessel 130, the third tube input port 1131, the third tube 113, the third tube fourth output sub-port 11324, the seventh tube 117, and the first fluid receiving component 171. That is, the primary cell sorting is implemented by allowing the sample fluid to flow along this fluid path. As the sample fluid passes through the sorting vessel 130, the target cells in the sample fluid are adsorbed by the sorting vessel 130, and the sample fluid that finally flows into the first fluid receiving component 171 contains components other than the target cells.

At step 3, the sorting vessel 130 is rinsed after the primary cell sorting is completed. The rinsing of the sorting vessel 130 after the primary cell sorting is completed is intended to remove non-target cells (e.g., cells that are not labeled with the magnetic particles) remaining in the tubes and the sorting vessel 130 after the primary cell sorting. A fluid path corresponding to the rinsing of the sorting vessel 130 is the same as the fluid path corresponding to the tube pre-rinsing. Accordingly, for how to rinse the sorting vessel 130, reference may be made to the related description of tube pre-rinsing.

At step 4, the target cells in the sorting vessel 130 are released. After the rinsing of the sorting vessel 130, the magnetic field acting on the sorting vessel 130 is removed such that the target cells adsorbed in the sorting vessel 130 flow from the sorting vessel 130 and circulate along a corresponding fluid path. In some embodiments, a fluid path corresponding to the release of the target cells from the sorting vessel 130 sequentially includes: the sorting vessel 130, the second open end 134 of the sorting vessel 130, the third tube input port 1131, the third tube 113, the third tube second output sub-port 11322, the fourth tube 114, the first tube first output sub-port 11121, the first tube second output sub-port 11122, the peristaltic hose 140, the second tube input port 1121, the second tube 112, the second tube output port 1122, the first open end 133 of the sorting vessel 130, and the sorting vessel 130. That is, releasing the target cells from the sorting vessel 130 is implemented by allowing the target cells in the sorting vessel 130 to circulate along this fluid path.

At step 5, secondary cell sorting is carried out. After the release of the target cells from the sorting vessel 130 is completed, the magnetic field is again applied to the sorting vessel 130 such that the target cells flow into the sorting vessel 130 from the second open end 134 of the sorting vessel 130 and the sorting vessel 130 adsorbs the target cells therein again under the action of the magnetic field. A fluid path corresponding to the secondary cell sorting sequentially includes: the first open end 133 of the sorting vessel 130, the second tube output port 1122, the second tube 112, the second tube input port 1121, the peristaltic hose 140, the first tube second output sub-port 11122, the first tube first output sub-port 11121, the fourth tube 114, the third tube second output sub-port 11322, the third tube 113, the third tube input port 1131, the second open end 134 of the sorting vessel 130, and the sorting vessel 130. That is, the secondary cell sorting is implemented by allowing the target cells to flow along this fluid path such that the sorting vessel 130 adsorbs the target cells therein again under the action of the magnetic field.

At step 6, the sorting vessel 130 is rinsed after the secondary cell sorting is completed. The rinsing of the sorting vessel 130 after the secondary cell sorting is completed is intended to remove non-target cells (e.g., cells that are not labeled with the magnetic particles) remaining in the tubes and the sorting vessel 130 after the secondary cell sorting. A fluid path corresponding to the rinsing of the sorting vessel 130 after the secondary cell sorting is completed is the same as the fluid path corresponding to the rinsing the sorting vessel 130 after the tube pre-rinsing and/or the primary cell sorting is completed. Accordingly, for how to rinse the sorting vessel 130 again, reference may be made to the related description of the rinsing the sorting vessel 130 after the tube pre-rinsing and/or the primary cell sorting is completed.

At step 7, the target cells are collected. After the rinsing of the sorting vessel 130 after the secondary cell sorting is completed, the magnetic field acting on the sorting vessel 130 is removed such that the target cells adsorbed in the sorting vessel 130 flow to the third fluid receiving component 173 for collection. A fluid path corresponding to the collection of the target cells sequentially includes: the second fluid supply component 162, the sixth tube 116, the first tube second input sub-port 11112, the first tube 111, the first tube second output sub-port 11122, the peristaltic hose 140, the second tube input port 1121, the second tube 112, the second tube output port 1122, the first open end 133 of the sorting vessel 130, the sorting vessel 130, the second open end 134 of the sorting vessel 130, the third tube input port 1131, the third tube 113, the third tube first output sub-port 11321, the ninth tube 119, and the third fluid receiving component 173. That is, the collection of the target cells involves allowing the buffer fluid in the second fluid supply component 162 to flow along this fluid path to flush the target cells in the sorting vessel 130 out of the second open end 134 of the sorting vessel 130 and into the third fluid receiving component 173 for collection. After the collection of the target cells is completed, the entire cell sorting process is completed.

In some embodiments, the tube assembly 100 may further comprise a filter vessel, and two ends of the filter vessel are connected to the second tube input port and the second tube output port, respectively. The filter vessel may capture non-target cell components of the sample fluid that may be adsorbed by the sorting vessel 130 before the sample fluid flows into the sorting vessel 130 during the primary cell sorting, so as to ensure that the sample fluid flowing into the sorting vessel 130 contain no or fewer non-target cell components that can be adsorbed by the sorting vessel 130, thereby ensuring a high purity of the sorted target cells, and accordingly improving the efficiency and purity of cell sorting. In some embodiments, the filter vessel and the sorting vessel may have the same or similar structure. When the sample fluid flows into the filter vessel, the filter vessel may capture the non-target cell components of the sample fluid that can be adsorbed by the sorting vessel 130 in the absence of the magnetic field. For the related description of the filter vessel, reference may be to the description of the sorting vessel 130 in FIG. 5.

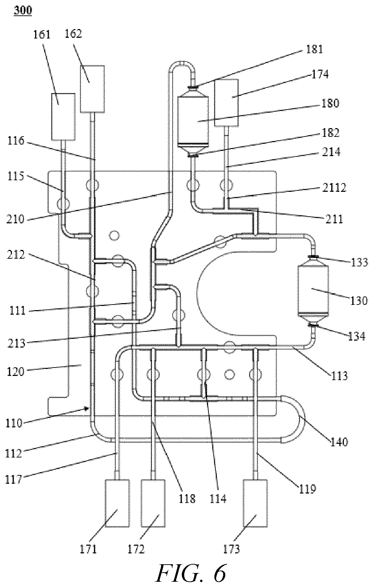

The tube assembly comprising the filter vessel will be described in detail below with respect to the accompanying drawings. FIG. 6 is a schematic structural diagram of a tube assembly comprising a filter vessel according to some embodiments of this specification. It should be noted that a tube assembly 200 shown in FIG. 6 is a variant improved based on the tube assembly 100 shown in FIG. 1, and therefore, components (e.g., the first tube 111, the second tube 112, and the third tube 113, etc.) of the tube assembly 200 that are the same as those of the tube assembly 100 are provided with the same reference numerals. Reference may be made to the related description of the tube assembly 100 for the description of the components of the tube assembly 200 that are the same as those of the tube assembly 100, which will not be repeated herein.

As shown in FIG. 6, the tube assembly 200 comprises a filter vessel 180 in addition to all of the components of the tube assembly 100. The filter vessel 180 can be connected to the second tube output port 1122.

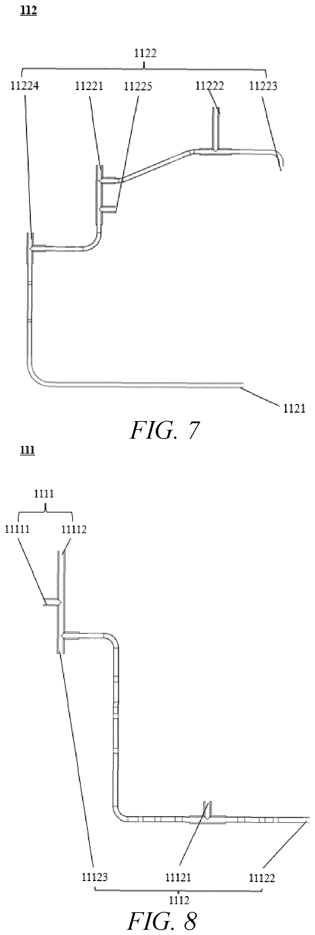

In some embodiments, the filter vessel 180 may not be included in the tube assembly 100, that is, the filter vessel 180 may be connected to the tube assembly 100 and mounted to the cell sorter independently of the tube assembly 100. In some embodiments, the filter vessel 180 may be connected to the tube assembly 100 and/or mounted to the cell sorter before or after the tube assembly 100 is mounted to the cell sorter. In some embodiments, the tube assembly 100 may additionally reserve at least two ports (e.g., a second tube first output sub-port 11221 and a second tube second output sub-port 11222 shown in FIG. 7) for connection with two ends of the filter vessel 180.

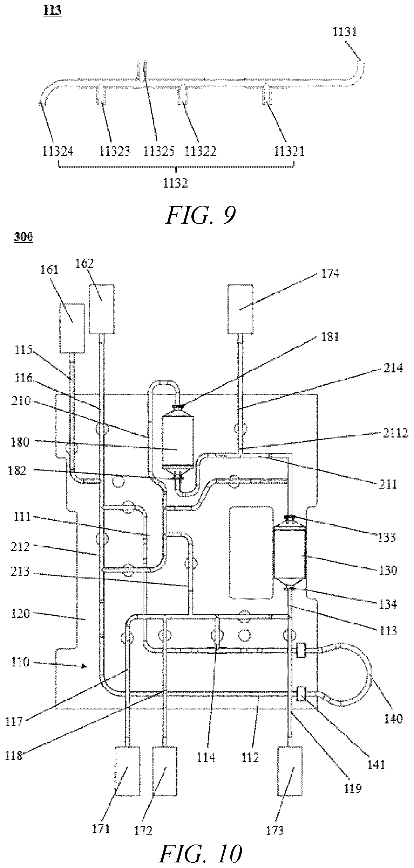

FIG. 7 is a schematic structural diagram of a second tube of the tube assembly shown in FIG. 6. FIG. 8 is a schematic structural diagram of a first tube of the tube assembly shown in FIG. 6. FIG. 9 is a schematic structural diagram of a third tube of the tube assembly shown in FIG. 6.

In some embodiments, as shown in FIG. 7, the second tube output port 1122 may comprise a second tube first output sub-port 11221, a second tube second output sub-port 11222, and a second tube third output sub-port 11223. With reference to FIG. 6, the fluid tubes 110 may further comprise a tenth tube 210 and an eleventh tube 211. Two ends of the tenth tube 210 are connected to the second tube first output sub-port 11221 and a first open end 181 of the filter vessel 180, respectively. Two ends of the eleventh tube 211 are connected to a second open end 182 of the filter vessel 180 and the second output sub-port 11222 of the second tube, respectively. The second tube third output sub-port 11223 is connected to the first open end 133 of the sorting vessel 130.

In some embodiments, with continued reference to FIG. 7, the second tube output port 1122 may further comprise a second tube fourth output sub-port 11224 and a second tube fifth output sub-port 11225. As shown in FIG. 8, the first tube output port 1112 may further comprise a first tube third output sub-port 11123. As shown in FIG. 9, the third tube output port may further comprise a third tube fifth output sub-port 11325. With reference to FIG. 6, the fluid tubes 110 further comprise a twelfth tube 212 and a thirteenth tube 213. Two ends of the twelfth tube 212 are connected to the second tube fourth output sub-port 11224 and the first tube third output sub-port 11123, respectively; and two ends of the thirteenth tube 213 are connected to the second tube fifth output sub-port 11225 and the third tube fifth output sub-port 11325, respectively.

In some embodiments, as shown in FIG. 6, the tube assembly 200 may further comprise a fourth fluid receiving component 174. The eleventh tube 211 is provided with an eleventh tube output port 2112. The fluid tubes 110 further comprise a fourteenth tube 214. Two ends of the fourteenth tube 214 are connected to the fourth fluid receiving component 174 and the eleventh tube output port 2112, respectively.

By adding the second tube fourth output sub-port 11224, the second tube fifth output sub-port 11225, the first tube third output sub-port 11123, the twelfth tube 212, the thirteenth tube 213, the fourteenth tube 214, the eleventh tube output port 2112 and the fourth fluid receiving component 174, the length of a corresponding fluid path in a corresponding step (e.g., tube pre-rinsing) of the cell sorting process can be reduced, thereby reducing the time for flow of the fluid in that step and improving the cell sorting efficiency.

In some embodiments, in the tube assembly 200, the fluid tubes 110 form a plurality of fluid paths for cell sorting among the sorting vessel 130, the filter vessel 180, and the peristaltic hose 140, and each of the plurality of fluid paths passes through a sorting column and the peristaltic hose for implementing the various steps of the cell sorting process. The cell sorting process will be described in detail below with respect to the tube assembly 200 shown in FIG. 6.

In some embodiments, the cell sorting process based on the tube assembly 200 may include the following steps.

At step 1, the tubes are pre-rinsed. The pre-rinsing of the tubes includes pre-rinsing the filter vessel 180 and pre-rinsing the sorting vessel 130. A fluid path corresponding to the pre-rinsing of the filter vessel 180 sequentially includes: the second fluid supply component 162, the first tube second input sub-port 11112, the first tube 111, the first tube second output sub-port 11122, the peristaltic hose 140, the second tube input port 1121, the second tube 112, the second tube first output sub-port 11221, the tenth tube 210, the first open end 181 of the filter vessel 180, the filter vessel 180, the second open end 182 of the filter vessel 180, the eleventh tube 211, the eleventh tube output port 2112, and the fourth fluid receiving component 174. That is, the buffer fluid flows along this fluid path to pre-rinse the filter vessel 180. A fluid path corresponding to the pre-rinsing of the sorting vessel 130 sequentially includes: the second fluid supply component 162, the first tube second input sub-port 11112, the first tube 111, the first tube third output sub-port 11123, the twelfth tube 212, the second tube fourth output sub-port 11224, the second tube 112, the second tube input port 1121, the peristaltic hose 140, the first tube second output sub-port 11122, the first tube first output sub-port 11121, the fourth tube 114, the third tube second output sub-port 11322, the third tube input port 1131, the second open end 134 of the sorting vessel 130, the sorting vessel 130, the first open end 133 of the sorting vessel 130, the second tube third output sub-port 11223, the second tube second output sub-port 11222, the eleventh tube 211, the eleventh tube output port 2112, and the fourth fluid receiving component 174. That is, the buffer fluid flows along this fluid path to pre-rinse the sorting vessel 130.

At step 2, primary cell sorting is carried out. A fluid path corresponding to the primary cell sorting sequentially includes: the first fluid supply component 161, the first tube first input sub-port 11111, the first tube 111, the first tube second output sub-port 11122, the peristaltic hose 140, the second tube input port 1121, the second tube 112, the second tube first output sub-port 11221, the tenth tube 210, the first open end 181 of the filter vessel 180, the filter vessel 180, the second open end 182 of the filter vessel 180, the eleventh tube 211, the second tube second output sub-port 11222, the second tube third output sub-port 11223, the first open end 133 of the sorting vessel 130, the sorting vessel 130, the second open end 134 of the sorting vessel 130, the third tube input port 1131, the third tube fourth output sub-port 11324, the seventh tube 117, and the first fluid receiving component 171. That is, the primary cell sorting is implemented by allowing the sample fluid to flow along this fluid path. For further description of the primary cell sorting, reference may be made to the related description of the primary cell sorting in the cell sorting process based on the tube assembly 100.

At step 3, the sorting vessel 130 is rinsed after the primary cell sorting. A fluid path corresponding to the rinsing of the sorting vessel 130 after the primary cell sorting sequentially includes: the second fluid supply component 162, the first tube second input sub-port 11112, the first tube 111, the first tube second output sub-port 11122, the peristaltic hose 140, the second tube input port 1121, the second tube 112, the second tube first output sub-port 11221, the tenth tube 210, the first open end 181 of the filter vessel 180, the filter vessel 180, the second open end 182 of the filter vessel 180, the eleventh tube 211, the second tube second output sub-port 11222, the second tube third output sub-port 11223, the first open end 133 of the sorting vessel 130, the sorting vessel 130, the second open end 134 of the sorting vessel 130, the third tube input port 1131, the third tube third output sub-port 11323, the eighth tube 118, and the second fluid receiving component 172. That is, the rinsing of the sorting vessel 130 after the primary cell sorting is implemented by allowing the buffer fluid to flow along the fluid path.

At step 4, the target cells in the sorting vessel 130 are released. A fluid path corresponding to the release of the target cells from the sorting vessel 130 sequentially includes: the sorting vessel 130, the second open end 134 of the sorting vessel 130, the third tube input port 1131, the third tube second output sub-port 11322, the fourth tube 114, the first tube first output sub-port 11121, the first tube second output sub-port 11122, the peristaltic hose 140, the second tube input port 1121, the second tube 112, the second tube first output sub-port 11221, the second tube third output sub-port 11223, the first open end 133 of the sorting vessel 130, and the sorting vessel 130. That is, releasing the target cells from the sorting vessel 130 is implemented by allowing the target cells in the sorting vessel 130 to circulate along this fluid path.

At step 5, secondary cell sorting is carried out. A fluid path corresponding to the secondary cell sorting sequentially includes: the first open end 133 of the sorting vessel 130, the second tube third output sub-port 11223, the second tube 112, the second tube input port 1121, the peristaltic hose 140, the first tube second output sub-port 11122, the first tube first output sub-port 11121, the fourth tube 114, the third tube second output sub-port 11322, the third tube input port 1131, the second open end 134 of the sorting vessel 130, and the sorting vessel 130. That is, the secondary cell sorting is implemented by allowing the target cells to flow along the fluid path into the sorting vessel 130 such that the sorting vessel 130 adsorbs the target cells therein under the action of the magnetic field.

At step 6, the sorting vessel 130 is rinsed after the secondary cell sorting. A fluid path corresponding to the rinsing of the sorting vessel 130 after the secondary cell sorting is the same as the fluid path corresponding to the rinsing of the sorting vessel 130 after the primary cell sorting, which will not be repeated herein.

At step 7, the target cells are collected. A fluid path corresponding to the collection of the target cells sequentially includes: the second fluid supply component 162, the first tube second input sub-port 11112, the first tube 111, the first tube third output sub-port 11123, the twelfth tube 212, the second tube fourth output sub-port 11224, the second tube 112, the second tube input port 1121, the peristaltic hose 140, the first tube second output sub-port 11122, the first tube first output sub-port 11121, the fourth tube 114, the third tube second output sub-port 11322, the third tube 113, the third tube fifth output sub-port 11325, the thirteenth tube 213, the second tube fifth output sub-port 11225, the second tube third output sub-port 11223, the first open end 133 of the sorting vessel 130, the sorting vessel 130, the second open end 134 of the sorting vessel 130, the third tube input port 1131, the third tube first output sub-port 11321, the ninth tube 119, and the third fluid receiving component 173. That is, with the magnetic field acting on the sorting vessel 130 being removed, the target cells are collected by allowing the buffer fluid to flow along this fluid path to flush the target cells out of the sorting vessel 130 and into the third fluid receiving component 173.

In some embodiments, a plurality of mounting grooves may be provided on one side of the mounting plate of the tube assembly 100 or 200 provided in the embodiments of this specification. The plurality of mounting grooves may be configured for fixedly mounting and/or guiding the fluid tubes 110, making the fluid tubes 110 concise and neat. For example, in the tube assembly 100, nine mounting grooves may be provided on one side of the mounting plate 120. The nine mounting grooves may be configured for mounting the first tube 111, the second tube 112, the third tube 113, the fourth tube 114, the fifth tube 115, the sixth tube 116, the seventh tube 117, the eighth tube 118 and the ninth tube 119 of the tube assembly 100, respectively. As another example, in the tube assembly 200, fourteen mounting grooves may be provided on one side of the mounting plate 120. The fourteen mounting grooves may be configured for mounting the first tube 111, the second tube 112, the third tube 113, the fourth tube 114, the fifth tube 115, the sixth tube 116, the seventh tube 117, the eighth tube 118, the ninth tube 119, the tenth tube 210, the eleventh tube 211, the twelfth tube 212, the thirteenth tube 213 and the fourteenth tube 214 of the tube assembly 200, respectively. In some embodiments, the plurality of mounting grooves may be connected to one another to facilitate interconnection between the tubes after the tubes are mounted in the mounting grooves. In some embodiments, the mounting grooves shaped to adapt to the tubes corresponding thereto, such that the tubes can be quickly and accurately mounted into corresponding mounting grooves. In some embodiments, the fluid tubes 110 may be composed of a plurality of hoses. The plurality of hoses may be mounted into corresponding mounting grooves and guided (or constrained) by the mounting grooves to be formed in the forms of the corresponding tubes.

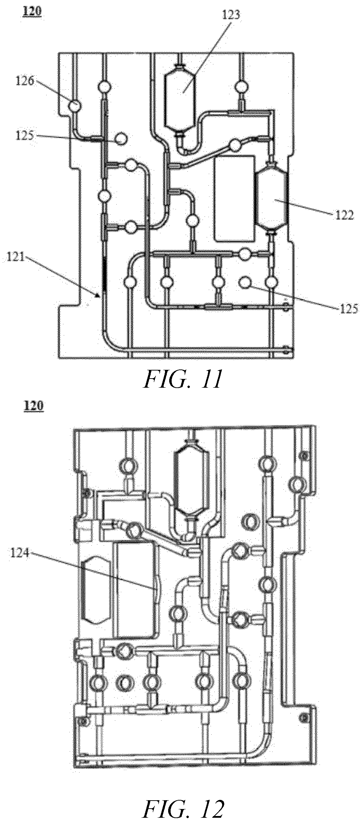

In some embodiments, at least one of the plurality of mounting grooves can be configured for mounting the filter vessel 180 and/or the sorting vessel 130 such that the filter vessel 180 and/or the sorting vessel 130 can be mounted to the mounting plate 120 along with the fluid tubes 110. For example, in the tube assembly 100, the mounting plate 120 may be provided with a mounting groove for mounting the sorting vessel 130. As another example, in the tube assembly 200, mounting grooves for mounting the sorting vessel 130 and the filter vessel 180 may be provided in the mounting plate 120. FIG. 10 is a schematic structural diagram of a tube assembly according to some embodiments of this specification. FIG. 11 is a front schematic structural diagram of a mounting plate of the tube assembly shown in FIG. 10. FIG. 12 is a back schematic structural diagram of the mounting plate of the tube assembly shown in FIG. 10. It should be noted that the tube assembly 300 shown in FIG. 10 has the same fluid tube structure as the tube assembly 200 shown in FIG. 6, and accordingly the same reference numerals are used in FIG. 10 and FIG. 6. For the description of how to implement cell sorting with the tube assembly 300, reference may be made to the related description of the tube assembly 200.

As shown in FIGS. 10 and 11, each of the sorting vessel 130, the filter vessel 180 and the fluid tubes 110 are mounted to the mounting plate 120. The mounting plate 120 is provided with a sorting vessel mounting groove 122 and a filter vessel mounting groove 123 for mounting the sorting vessel 130 and the filter vessel 180, respectively, in addition to mounting grooves 121 for mounting the fluid tubes. With such an arrangement, the sorting vessel 130, the filter vessel 180 and the mounting plate 120 may be mounted to the cell sorter as a whole during cell sorting, such that the mounting steps for mounting the sorting vessel 130 and the filter vessel 180 are reduced and mounting time is saved. In addition, since the mounting groove 123 is provided for mounting the filter vessel 180, it can be ensured that the filter vessel 180 is kept upright without an additional mechanism during the cell sorting process, such that the non-target cell components of the sample fluid that can be adsorbed by the sorting vessel 130 can be captured as much as possible when the sample fluid flows through the filter vessel 180.

In some embodiments, as shown in FIG. 10, the peristaltic hose 140 can be mounted to the mounting plate 120 by retainer rings 141, and correspondingly, the mounting plate 120 is provided with mounting grooves for mounting the retainer rings 141 (not shown). The peristaltic hose 140 can be quickly and accurately mounted by mounting the retainer rings 141 into corresponding mounting grooves.

In some embodiments, for the tube assembly 100, 200 or 300 provided in the embodiments of this specification, a plurality of reinforcing ribs (e.g., reinforcing ribs 124 shown in FIG. 12) may be provided on the other side of the mounting plate 120 (i.e., a backside of the mounting plate 120) in order to enhance the strength and stiffness of the mounting plate 120.

In some embodiments, at least two asymmetrical positioning holes (e.g., positioning holes 125 shown in FIG. 11) are also provided in the mounting plate 120. The at least two asymmetric positioning holes may be fitted with a positioning mechanism on the cell sorter in order to prevent errors in mounting the tube assembly (e.g., the tube assembly 100, 200, or 300) to the cell sorter.

In some embodiments, a plurality of valve holes (e.g., valve holes 126 shown in FIG. 11) are provided in the mounting plate 120, and may be arranged in the plurality of mounting grooves. In some embodiments, when the tube assembly is mounted to the cell sorter, a plurality of valve assemblies (e.g., on-off valves) on the cell sorter may be fitted with the plurality of valve holes such that the valve assemblies may be jointed to the tubes mounted in the mounting grooves where the corresponding valve holes are located, thereby controlling on and off of the corresponding tubes to create fluid paths corresponding to the various steps of the cell sorting process.

In some embodiments, the tube assembly (e.g., the tube assembly 100, 200, or 300) further comprises on-off valves (not shown) disposed in the valve holes 126. A notch is provided at an end of each of the on-off valves facing an opening of the mounting groove. When the tubes of the fluid tubes 110 are mounted into the mounting grooves, the tubes are mounted in the notches of the on-off valves in the valve holes of the corresponding mounting grooves, such that the on-off valves control on and off of the corresponding tubes to create fluid paths corresponding to the various steps of the cell sorting process.

In some embodiments, the number of the valve holes of the mounting plate 120 may correspond to the number of the mounting grooves (or the tubes).

In some embodiments, in the tube assembly 100, the mounting plate 120 may comprise nine valve holes, such that corresponding valve assemblies (or on-off valves) can be provided to control on/off of the first tube 111, the second tube 112, the third tube 113, the fourth tube 114, the fifth tube 115, the sixth tube 116, the seventh tube 117, the eighth tube 118, and the ninth tube 119 respectively, so as to create corresponding fluid paths. In the case of the fluid path corresponding to the step of tube pre-rinsing of the cell sorting process based on the tube assembly 100, the fluid path corresponding to the step of tube pre-rinsing can be created by opening the valve assemblies (or on-off valves) corresponding to the sixth tube 116, the first tube 111, the second tube 112, the third tube 113 and the eighth tube 118 and closing the other tubes (e.g., the fourth tube 114, the fifth tube 115, the sixth tube 116, the seventh tube 117 and the ninth tube 119).

In some embodiments, in the tube assembly 200, the mounting plate 120 may comprise 14 valve holes, such that corresponding valve assemblies (or on-off valves) can be provided to control on/off of the first tube 111, the second tube 112, the third tube 113, the fourth tube 114, the fifth tube 115, the sixth tube 116, the seventh tube 117, the eighth tube 118, the ninth tube 119, the tenth tube 210, the eleventh tube 211, the twelfth tube 212, the thirteenth tube 213, and the fourteenth tube 214, respectively, so as to create corresponding fluid paths. For the description of how to create a fluid path corresponding to each step of the cell sorting process based on the tube assembly 200, reference may be made to the creation of the fluid path corresponding to the step of tube pre-rinsing of the cell sorting process based on the tube assembly 100.

In some embodiments, it may not be required for the on-off valves to control on/off of some of the tubes in the tube assembly, but the creation of the fluid paths corresponding to the various steps of the cell sorting process by the tube assembly can also be ensured, and accordingly, the number of the valve holes in the mounting plate 120 may be smaller than the number of the mounting grooves. For example, the mounting plate 120 shown in FIG. 1 may comprise seven valve holes, and the number of mounting grooves is nine, where the valve holes may not be provided in the mounting grooves corresponding to the first tube 111 and the third tube 113. As another example, the mounting plate 120 shown in FIG. 6 may comprise thirteen valve holes, and the number of mounting grooves is fourteen, where no valve holes may be provided in the mounting groove corresponding to the tenth tube 210.

The possible beneficial effects of the embodiments of this specification include but are not limited to the following aspects: (1) according to the tube assembly in the embodiments of this specification, the fluid tubes and/or the sorting vessel and/or the filter vessel are mounted to the mounting plate as a whole, and then are mounted to the cell sorter, and an operator can quickly and accurately mount the tube assembly to the cell sorter, thereby saving on time and labor and reducing the possibility of mistakes or errors in manual mounting; (2) the reinforcing ribs are provided on the mounting plate, which can increase the strength and stiffness of the mounting plate; (3) a plurality of mounting grooves are provided in the mounting plate to facilitate mounting and/or guiding of the fluid tubes and/or the sorting vessel and/or the filter vessel, which makes the entire tube assembly compact, simple and aesthetic; (4) a plurality of valve holes are provided in the mounting plate facilitate fitting of the valve assemblies (or on-off valves) therein to control on/off of corresponding tubes so as to create a plurality of fluid paths for cell sorting; (5) the mounting groove is provided in the mounting plate to mount the filter vessel, it can be ensured that the filter vessel is kept upright without an additional mechanism, thus enabling the filter vessel to have a filtering effect; and (6) the mounting grooves are provided in the mounting plate to mount the retainer rings for the peristaltic hose, facilitating the mounting of the peristaltic hose.

It should be noted that different embodiments may have different beneficial effects, and the beneficial effects that may be produced in the different embodiments may be any one or a combination of the above, or may be any other beneficial effects that may be obtained.

The basic concepts have been described above, and it will be apparent to those skilled in the art that the foregoing detailed disclosure is intended to be exemplary only and does not constitute a limitation to the present application. Although not explicitly stated herein, those skilled in the art may make various modifications, improvements and amendments to the present application. Such modifications, improvements and amendments are suggested in the present application, and thus remain within the spirit and scope of the exemplary embodiments of the present application.

Meanwhile, the present application uses specific words to describe the embodiments of the present application. For example, "one embodiment", "an embodiment" and/or "some embodiments" refers to a particular feature, structure or characteristic related to at least one embodiment of the present application. Hence, it should be emphasized and noted that "an embodiment" or "one embodiment" or "one alternative embodiment" mentioned two or more times in different positions in this specification does not necessarily refer to the same embodiment. Furthermore, some features, structures or characteristics in one or more embodiments of the present application may be appropriately combined.

Furthermore, unless explicitly stated in the claims, the order of processing elements and sequences, the use of numbers and letters, or the use of other names described in the present application is not intended to limit the order of the process or method of the present application. Although some currently considered useful embodiments of the invention have been discussed in the above disclosure by way of various examples, it should be understood that such details are only for illustrative purposes, and that the appended claims are not limited to the disclosed embodiments; rather, the claims are intended to cover all amendments and equivalent combinations that are in tube with the spirit and scope of the embodiments of the present application. For example, although the system components described above can be implemented by means of hardware devices, they may also be implemented only by software solutions, such as installing the described system on an existing server or mobile device.

Similarly, it should be noted that in order to simplify the presentation of the disclosure of the present application, and thereby help to understand one or more embodiments of the invention, in the previous descriptions of the embodiments of the present application, various features are sometimes combined into one embodiment, the accompanying drawings, or descriptions thereof. However, this method of disclosure does not imply that the subject of the present application requires more features than those mentioned in the claims. In fact, the embodiments have fewer features than all features of the individual embodiments disclosed above.

In some embodiments, numbers for describing components, properties and quantities are used. It should be understood that such numbers for describing the embodiments are modified by the modifiers "about", "approximately" or "substantially" in some examples. Unless otherwise stated, the modifiers "about", "approximately" or "substantially" indicate that the number is allowed to vary by ± 20%. Accordingly, in some embodiments, numerical parameters used in the specification and claims are approximate values that may vary depending on desired features of individual embodiments. In some embodiments, with regard to the numerical parameters, the specified number of significant digits should be taken into account, and a general digit retaining method should be used. Although numerical ranges and parameters for determining the breadth of ranges in some embodiments of the present application are approximate values, such numerical values should be set as accurate as possible in feasible ranges in the specific embodiments.

Each patent, each patent application, each patent application publication and other materials, such as articles, books, instructions, publications, documents, etc. cited in the present application are hereby incorporated by reference into the present application in its entirety. Application history documents that are inconsistent with or conflict with the contents of the present application are excluded, and documents (currently or later appended to the present application) that limit the broadest scope of the claims of the present application are also excluded. It should be noted that if there is any inconsistency or conflict between the descriptions, definitions, and/or the use of terms in the accompanying materials of the present application and the contents of the present application, the descriptions, definitions, and/or the use of terms in the present application shall prevail.

Finally, it should be understood that the embodiments described in the present application are only intended to illustrate the principles of the embodiments of the present application. Other variations may also fall within the scope of the present application. Thus, by way of example but not limitation, alternative configurations of the embodiments of the present application can be considered consistent with the teachings of the present application. Accordingly, the embodiments of the present application are not limited to those explicitly presented and described herein.

## Claims

1. A tube assembly, **characterized by** comprising fluid tubes and a mounting plate for mounting at least the fluid tubes; wherein
the fluid tubes comprise at least a first tube, a second tube, and a third tube, wherein
the first tube comprises a first tube input port and a first tube output port, the first tube input port comprising at least a first tube first input sub-port and a first tube second input sub-port, and the first tube output port comprising at least a first tube first output sub-port and a first tube second output sub-port;
the second tube comprises a second tube input port and a second tube output port; and
the third tube comprises a third tube input port and a third tube output port, the third tube output port comprising a third tube first output sub-port, a third tube second output sub-port, a third tube third output sub-port, and a third tube fourth output sub-port.

2. The tube assembly according to claim 1, **characterized in that** the fluid tubes further comprise a fourth tube, two ends of the fourth tube being connected to the first tube first output sub-port and the third tube second output sub-port, respectively.

3. The tube assembly according to claim 1, **characterized in that** the tube assembly further comprises a sorting vessel, a first open end and a second open end of the sorting vessel being connected to the second tube output port and the third tube input port, respectively.

4. The tube assembly according to claim 1, **characterized in that** the tube assembly further comprises a peristaltic hose, two ends of the peristaltic hose being connected to the first tube second output sub-port and the second tube input port, respectively.

5. The tube assembly according to claim 1, **characterized in that** the tube assembly comprises at least one fluid supply component, the at least one fluid supply component connected to the first tube input port of the first tube.

6. The tube assembly according to claim 5, **characterized in that** the at least one fluid supply component comprises a first fluid supply component and a second fluid supply component; and the fluid tubes further comprise a fifth tube and a six tube; wherein
two ends of the fifth tube are connected to the first fluid supply component and the first tube first input sub-port, respectively; and
two ends of the sixth tube are connected to the second fluid supply component and the first tube second input sub-port, respectively.

7. The tube assembly according to claim 1, **characterized in that** the tube assembly comprises at least one fluid receiving component, the at least one fluid receiving component connected to the third tube output port of the third tube.

8. The tube assembly according to claim 7, **characterized in that** the at least one fluid receiving component comprises a first fluid receiving component, a second fluid receiving component, and a third fluid receiving component; and the fluid tubes further comprise a seventh tube, an eighth tube, and a ninth tube; wherein
two ends of the seventh tube are connected to the first fluid receiving component and the third tube fourth output sub-port, respectively;
two ends of the eighth tube are connected to the second fluid receiving component and the third tube third output sub-port, respectively; and
two ends of the ninth tube are connected to the third fluid receiving component and the third tube first output sub-port, respectively.

9. The tube assembly according to any one of claims 1-8, **characterized in that** the tube assembly further comprises a filter vessel, the filter vessel connected to the second tube output port.

10. The tube assembly according to claim 9, **characterized in that** the second tube output port comprises a second tube first output sub-port, a second tube second output sub-port, and a second tube third output sub-port; and the fluid tubes further comprise a tenth tube and an eleventh tube; wherein
two ends of the tenth tube are connected to the second tube first output sub-port and a first open end of the filter vessel, respectively;
two ends of the eleventh tube are connected to a second open end of the filter vessel and the second tube second output sub-port, respectively; and
the second tube third output sub-port is connected to the first open end of the sorting vessel.

11. The tube assembly according to claim 10, **characterized in that** the second tube output port further comprises a second tube fourth output sub-port and a second tube fifth output sub-port; the first tube output port further comprises a first tube third output sub-port; the third tube output port further comprises a third tube fifth output sub-port; and the fluid tubes further comprise a twelfth tube and a thirteenth tube; wherein
two ends of the twelfth tube are connected to the second tube fourth output sub-port and the first tube third output sub-port, respectively; and
two ends of the thirteenth tube are connected to the second tube fifth output sub-port and the third tube fifth output sub-port, respectively.

12. The tube assembly according to claim 10, **characterized in that** the tube assembly further comprises a fourth fluid receiving component; an eleventh tube output port is provided in the eleventh tube; and the fluid tubes further comprise a fourteenth tube, wherein
two ends of the fourteenth tube are connected to the fourth fluid receiving component and the eleventh tube output port, respectively.

13. The tube assembly according to claim 12, **characterized in that** a plurality of mounting grooves are provided on one side of the mounting plate, and the plurality of mounting grooves are configured for fixedly mounting and/or guiding the fluid tubes.

14. The tube assembly according to claim 13, **characterized in that** at least one of the plurality of mounting grooves is configured for fixedly mounting the filter vessel and/or the sorting vessel.

15. The tube assembly according to claim 13, **characterized in that** the mounting plate is provided with a plurality of reinforcing ribs on a side opposite the mounting grooves.

16. The tube assembly according to claim 13, **characterized in that** at least two asymmetrical positioning holes are provided in the mounting plate.

17. The tube assembly according to claim 13, **characterized in that** a plurality of valve holes are provided in the mounting plate, and are arranged in the plurality of mounting grooves.

18. The tube assembly according to claim 17, **characterized in that** the tube assembly further comprises on-off valves disposed in the valve holes, a notch being provided at an end of each of the on-off valves facing an opening of the mounting groove and being configured for mounting a tube of the fluid tubes.

19. The tube assembly according to claim 12, **characterized in that** the fluid tubes form a plurality of fluid paths among the sorting vessel and/or the filter vessel and the peristaltic hose, and each of the plurality of fluid paths passes through the sorting column and the peristaltic hose.
